Europäisches Patentamt

European Patent Office

Office européen des brevets

(11) Publication number: **0 367 549**
**A2**

# EUROPEAN PATENT APPLICATION

(21) Application number: **89311212.8**

(22) Date of filing: **31.10.89**

(51) Int. Cl.5: **A61M 39/02**

(30) Priority: **31.10.88 US 264533**

(43) Date of publication of application:
**09.05.90 Bulletin 90/19**

(84) Designated Contracting States:
**AT BE CH DE ES FR GB GR IT LI LU NL SE**

(71) Applicant: **Lynn, Lawrence A.**
**862 Curleys Court**
**Worthington Ohio 43085(US)**

(72) Inventor: **Lynn, Lawrence A.**
**862 Curleys Court**
**Worthington Ohio 43085(US)**

(74) Representative: **Laight, Martin Harvey et al**
**W H Beck, Greener & Company 7 Stone**
**Buildings Lincoln's Inn**
**London WC2A 3SZ(GB)**

(54) **Medical connector.**

(57) A medical connector comprises a first connecting portion (244) and a second connecting portion (266), and connects two fluid conveying conduits (150) and (120), one of which has a septum (152) at one end thereof. The second connecting means (266) is connected by a flange (267) to a syringe (26), and has a needle (270) extending into a space bounded by flexible fingers (248). The septum (152) of a further conduit (150) is pushed into the space bounded by the fingers (248) so that the needle (270) penetrates the septum (152). An outer tube (242) is pushed manually over the fingers (248) flexing them inwardly to trap and lock the septum (152).

FIG. 30.

## MEDICAL CONNECTOR

The attachment of intravenous tubing to intravascular catheters for the administration of fluids and or medication to patients has been widely utilized for over three decades. Generally, an intravenous tubing system comprises a long segment of tubing which is proximally attached to an elevated bag or bottle of fluid and distally attached to an intravascular catheter. Such a system acts as a primary system, and a secondary conduit may be connected to the primary system. To allow for such connection, such primary systems often have a "y" shaped branch junction section with a penetrable self-sealing septum. A needle attached to the end of the second conduit can be inserted through such a septum to create fluid connection between the primary system and second conduit. Thus, the septum provides a mechanism for the recurrent and frequent connection of secondary, "piggyback" intravenous tubing systems to the primary tubing system. Such second or piggyback systems typically include a small bag of antibiotic or other medication which is dissolved in a fluid and connected to a long flexible plastic conduit. The intermittent connections of such secondary intravenous systems to the primary system allows the administration of the medication at frequent intervals without disconnecting the primary intravenous system and without discontinuing fluid flow through the primary system.

Such "y" shaped tubes typically have a tubular trunk with a cylindrical tubular arm branching from it at an angle which can range from about 30° to 45°. The longitudinal section through such an arm is of elliptical shape. The major axis of this elliptical section varies with the angle of extension of the arm. In particular, the major axis length increases as the angle of arm extension decreases.

A major problem with such systems is that the needles frequently loosen and become disconnected from the septum during fluid administration. This can result in the medication spilling into the patient's bed or on the floor. An even greater problem is that the needle may become contaminated during disconnection. The contaminated needle portion may be readvanced through the septum, thereby introducing contamination into the primary tubing system.

U.S. Patent 4,752,292 discloses a device which helps prevent disconnection of a needle from a compatible interfacing tube having a septum. However, application of this device is limited to tubing systems with specifically compatible interfacing components which fit together. Therefore, the use of that disclosure would require structural changes to the conventional tubing systems which contain septae, and which are deployed in a wide range of existing tubing systems and have long been in use with heterogeneous populations of infusion pumping mechanisms and other devices.

There are, however, a considerable number of intravenous tubing systems which do not have a "Y" shaped junction section. These systems typically have a conduit which is occluded at its proximal end by a penetrable, self-sealing septum. Such tubing systems include heparin wells. These wells comprise a short segment of conduit commonly of rigid material such as polycarbonate. The segments are usually less than six centimeters in length and have a penetrable, self-sealing septum at their proximal end, and a female fitting for attachment to an indwelling venous catheter at a distal end.

These systems when attached to an indwelling catheter, comprise a single, short conduit segment with a blind proximal end occluded by a septum which can be filled with heparinized solution to maintain patency. Medications and fluid from secondary tubing systems can be connected to this heparin well conduit. This can be done by inserting a needle connected to the secondary tubing system through the septum at the proximal end of the heparin well to establish liquid connection between the secondary tubing system and the heparin well, and hence to the catheter and vascular system of the patient.

In addition to heparin wells, other conduits which are connectable to a patient's vascular system can have a penetrable self-sealing septum occluding the proximal end. Multi-lumen catheters for instance, commonly have multiple septae occluding the distal end of each different conduit which connects to the independent lumens of the multi-lumen catheter. Liquid connection into the patient's vasculature can be established by inserting a needle connected to a secondary tubing system through the septum occluding the proximal lumen end. Neither the heparin well nor the multi-lumen catheter commonly have the previously discussed Y-shaped junction sections.

According to the present invention in a first aspect there is provided a medical connector for connecting two fluid conveying conduits, comprising a first connecting portion adapted to be connected to a first fluid conveying conduit having a septum at an end thereof, and a second connecting portion adapted to be connected to a second fluid conveying conduit, characterised in that the second connecting portion has one end adapted to be connected to the second fluid conveying conduit and has at its other end a fluid conveying needle, and in that the first connecting portion defines a space into which the needle extends towards an open end of the space and which is adapted to receive the septum of the first fluid conveying conduit so that the needle penetrates the septum, the first connecting portion including flexible gripping means extending towards the said open end of the space and bounding the space, and there being providing locking means manually moveable from a first position in which the septum may be inserted into the said space through the said open end to be penetrated by the needle, to a second position where the locking means flexes the gripping means inwardly to trap and lock the septum in the space.

In one preferred form, the flexible gripping means comprise flexible fingers extending towards the open end of the said space.

In accordance with a preferred feature, the locking means extends around the first connecting portion and is slidable on the first connecting portion, or example the locking means may comprise a sleeve.

In accordance with another preferred feature, it is arranged that the needle is positioned in the said space at a location such as to reduce accessibility to manual contact by an operator.

In accordance with the invention in a second aspect there is provided a universal medical connector for manually connecting a first fluid conveying conduit in fluid connection with a patient's vasculature, and having a septum at an end thereof, to a second fluid conveying conduit in fluid connection with a source of fluid for administration to a patient, and having an open end thereof, comprising: means defining a needle hub having one end adapted to couple to said open end of said second fluid conveying conduit, the other end adapted for connection to a fluid conveying needle, and a bore extending therebetween for conveying fluid from an open ended conduit coupled to said one end to a needle connected to said other end; a single needle mounted to said other end of said needle hub; means defining a space through which said needle extends toward an open end thereof and adapted for receiving said septum of said first fluid conveying conduit so that said needle penetrates said septum and fluid flows through said needle into said first conduit, said needle being positioned in said space so as to be inaccessible to the fingers of a person connecting said conduits, said defining means further including flexible fingers extending toward said open end of said space and bounding said space; and locking means mounted about said space defining means and manually moveable from a first position in which said septum may be inserted into said space through said open end to be penetrated by said needle to a second position flexing said fingers inwardly to trap and lock said septum in said space.

In one arrangement said one end of said needle hub is provided with a flange surrounding the opening of said bore so that an open end of a second conduit can be releasably received on said flange or releasably received into said opening of said bore.

Conveniently said space defining means is a inner tube of molded plastic and said locking means is an outer tube of plastic and is more rigid than said inner tube. The said outer tube may be slidable along said inner tube in the direction said needle extends. The said inner tube may be provided with separated stop surfaces limiting the sliding movement of said outer tube.

In accordance with one preferred feature, said finger has a thickened portion adjacent the end thereof which flexes inwardly to lock said septum in said space.

The invention overcomes problems that exist in the art. In accordance with another aspect of the invention, relating to the "y" shaped junction arrangements which have been discussed hereinbefore, there may be provided a series of further independent, and/or related, features as follows. The invention may provide a member which can be mounted with the junction tube so that a secure relationship is established with the member and the arm of the "y" shaped junction section or tube to prevent inadvertent removal of the member from the junction tube. The member can have a conduit for extension into liquid flow connection with the junction tube. In an embodiment, the conduit can comprise a needle having a flow channel, with the needle being mounted with the member. When the member is mounted to the junction tube to lock with the arm, the needle can extend within the trunk of the junction tube.

The member further can have means for receiving liquid from the external liquid source. This means can comprise a receptacle associated with the tube which can be integral with the tube, or a separate component mounted with the tube, for example.

The means for establishing a secure relationship between the member and the arm of the junction tube can comprise a slot arrangement permitting insertion of the arm within the slot and movement of the member relative to the junction tube to move the arm into a locking relationship relative to a part of the slot. In an embodiment, the slot can comprise a "b" (lower case) shape having a longitudinal portion and an enlarged transverse portion or hollow slot. The longitudinal portion can have an opening at one end of the member. The member can be of a generally tubular shape.

The member can have a means to resist movement of the member relative to the arm to an unlocked position. This can comprise a projection into the slot to narrow the slot passageway to resist non-volitional movement of the arm relative to the slot. Yet the engagement permits the hand to move the arm to an unlocked position.

The slot portion which receives the arm has a portion of large enough breadth to accommodate receipt of the corresponding portion of the arm therethrough. When the arm is extending at an angle relative to the trunk, the corresponding cross section of the arm is of an elliptical shape. Hence, the slot portion that receives the arm in the locked position is of greater breadth than the major axis of the ellipse.

In an embodiment, the slot arrangement can be generally of a "b" shape on a cylindrical tube. In this embodiment, the width of the longitudinal portion of the slot can be less than the breadth of the transverse slot portion which receives the arm in the locking position.

The invention comprises the single member, although it is believed that a device comprising an ensemble of a first member and a second member is preferred. In the two member arrangement, the invention comprises a device having first member which can be mounted to the junction tube, and a second member which can be mounted with the first member. The first and second members have means for locking with the junction tube arm to prevent movement of the junction tube away from the second member.

One of the first or second members can have a means for receiving a liquid source for administration to a patient, such as from a separate syringe, or from another tube. The device also has means to provide flow of the liquid from the outside source to the junction tube.

In an embodiment of the invention, the first member and second member are both tubes with slots. The slots have parts that can be aligned so that the junction arm can be moved within the slots. One of the tubes has a slot with first and second sections so that the junction arm can be slid through the first slot section, and then the tube can be moved relative to the junction arm to have the second slot section extend about the junction arm. Such positioning blocks movement of the second tube relative to the junction arm in both longitudinal directions, and also blocks movement in a third transverse direction. The slot with two sections can be a general "b" shape. The transverse section of the "b" shaped slot provides an enlarged portion which is sized to accommodate the length of the major axis of the corresponding elliptical cross section of the arm.

Means for holding the arm within the locked position to resist non-volitional movement in the fourth direction can be provided. Such means can comprise a nib projecting from the wall of the second slot section to provide a resistance gate against passage of the arm. The nib can be of resilient material so that it flexes to allow the junction arm to pass to and from the second slot section when the first or second members are rotated by the hand, and likewise allows the junction arm to be rotated out of the second slot when the first or second members are hand rotated. This is also a means of preventing movement of the first and second tubes relative to each other, and of the junction tube relative to the first and second members by non-volitional movement.

When the tubes are rotated in relation to each other, the inner tube will remain in a fixed nonrotational relationship with the arm.

The two members can also have means to releasably engage each other when they are moved to be in a locking engagement with the junction arm, so that non-volitional movement of the first member relative to the second member to move those tubes to an unlocked position is resisted in the fourth direction. In one embodiment, the first and second members can have matching extensions and recesses which interfit to provide such releasable lock. In the embodiment using the tubes, the inner surface of the outer tube can have an extension or a recess which matches with a recess or an extension, respectively, on the outer surface of the inner tube. The tubes can be of resilient material to allow disengagement upon hand movement.

The two members can therefore be locked upon the arm with non-volitional movement being inhibited in all four directions, thereby preventing non-volitional disengagement of the two members from the arm.

The means for receiving a liquid source can be a receptacle located on one of the first or second members. The means for allowing liquid flow from the receptacle to within the junction tube can comprise a needle mounted to the device to pierce the septum on the junction tube. Fluid flow connection is provided between the receptacle and the needle to thus allow liquid flow from the receptacle into the junction tube.

A means for holding the first member against longitudinal movement relative to the second member is also provided. In an embodiment, one member can have a groove such as an annular groove, and the second member can have a projecting component that fits within the groove to allow the first tube to rotate relative to the second tube, but to prevent longitudinal movement of the first member relative to the second member. The projecting component can be an annular rim extending inwardly from the outer tube to fit within an annular groove of the inner tube. The projecting component can be flexible so that it can be enlarged by passage of the projection. In an em bodiment a frusto-conical shape connecting member is provided to force flexing of a resilient annular rim, with the rim flexing back to its initial position once it is received within the groove.

It is believed that the two member device provides greater stability and resistance against inadvertent disengagement than the single member arrangement. Further, the two member arrangement provides for stability despite the varying degrees of tightness of fit which results from accommodation of a wide range of angles for the arm relative to the trunk of the junction tube. For example, junction tube arms having smaller angles result in elliptical longitudinal sections having greater lengths of major axes at the intersection of the arm with the hollow. Therefore, such arms would fit only loosely into a hollow which is sized to accommodate junction sections of more acute angles. The addition of a second tube, therefore, provides for much greater stability since it has only a longitudinal slot which receives the branching angle into its longitudinal axis and therefore the required receiving width of the longitudinal slot is not affected by the branching angle. Therefore, a greater universal tightness of fit is secured throughout the conventionally used branching angles by an embodiment which comprises two tubes.

In another embodiment, the invention has an assembly that can lock about the proximal and connection terminal on a conduit, such as a septum, without relying on the "Y" shaped junction for the locking attachment. Such embodiment features the assembly having an outer member with a principal bore. That bore is sized to receive an inner elongated member. The inner member has a bore that receives the connection terminal, such as the septum. A needle can be mounted with the assembly to pierce the septum. Means are provided to cause the bore of the inner member to grip about the septum.

More specifically, as to one embodiment, the outer member and the inner member are both tubes. The inner tube is resilient and has longitudinal slots extending from its distal end toward its proximal end. The distal portion of the tubular wall of the inner tube can further have regions, which change in width, for interaction with the outer tube. The outer tube has projections that extend into the outer tube bore. These projections interact with the inner tube when the two tubes are moved relative to each other so that the projections abut the inner tube regions of varying width. The projections resultingly force the regions to grip about the septum.

In an embodiment, the regions have progressively increasing widths in a circumferential direction from one slot to another. The outer tube projections interact with the regions to narrow the inner tube distal bore.

In another embodiment, the wall of the inner tube has a portion of increased width near the distal end of the tube. The outer tube can interact with the portion of greater thickness to narrow the inner tube distal bore.

The invention thus also provides a means to attach to existing conventional septae which are presently in wide use, without the need for structural modifications to the conduit systems attached to the septae. The invention provides a universal fit by providing a means to tightly attach to septae of differing diameters.

Hence, in addition to the foregoing objects, it is also generally an object of the inventions to provide a means for secure connection of a secondary tubing system or fluid source to a "y" shaped junction section of a primary tubing system, as well as to sections not employing a "Y" shaped septum, without significant risk of inadvertent disconnection and contamination, or of accidental needle stick.

It is another object of the invention to provide a device which is completely suitable for use in conjunction with the "y" shaped junction sections deployed in the heterogeneous population of existing tubing systems, and which does not require any additions or changes to the structures of currently existing tubing systems. In particular, it is further the object of this invention to provide a device which can reliably lock onto a variety of conventional "y" shaped branch junction sections having variable branching angles and diameters.

It is further an object to provide a device that can releasably lock onto a variety of conventional straight junction sections having an connection terminal such as a septum.

These and other objects of the invention shall become apparent from the following description given by way of example , with reference to the drawings, in which:-

Figure 1 is a front elevation view showing the coupling device mounted to a junction tube, with a syringe connected to the coupling device;

Figure 2 is an exploded elevation view of the coupling device, showing toward the bottom the inner mount tube, and above the outer mount tube;

Figure 3 is an offset isometric view showing the outer mount tube and the inner mount tube;

Figure 4 is an elevation showing the coupling device mounted to the junction tube, with the outer mount tube mounted on the inner mount tube and rotated to the locked position;

Figure 5 is a section on the Line 5-5 of Figure 4;

Figure 6 is a top plan view taken on the Line 6-6 of Figure 5;

Figure 7 is a section of the coupling device and junction tube taken on the Line 7-7 of Figure 4;

Figure 8 is a section of the coupling device and junction tube taken on the Line 8-8 of Figure 4;

Figure 9 is a side elevation showing the coupling device mounted to the junction tube, in the unlocked position;

Figure 10 is a section of the coupling device and junction tube taken on the Line 10-10 of Figure 9;

Figure 11 is a section of the coupling device and mount tube taken on the Line 11-11 of Figure 9;

Figure 12 is an exploded isometric view of a modified coupling device having an inner and an outer mount tube; and

Figure 13 is a section view of the modification of Figure 12 shown mounted to a junction tube.

Figure 14 is a front elevation of a modification of the invention showing a modified coupling device mounted to a straight tube having an occluding septum, with the device in the locked position;

Figure 15 is a front plan exploded view of the modified coupling assembly of Figure 14;

Figure 16 is a front plan view of the modified coupling assembly, with the inner tube rotated relative to the outer tube to be in an unlocked position;

Figure 17 is an exploded isometric view of the modified coupling assembly;

Figure 18 is a section taken on the line 18-18 of Figure 14, with the device shown in the locked position;

Figure 19 is a section taken on the line 19-19 of Figure 18;

Figure 20 is a section similar to the section taken on the line 19-19, except showing the inner tube and outer tube in an unlocked position;

Figure 21 is a longitudinal section of the outer tube, with the inner hub and needle not shown in section;

Figure 22 is a view of the bottom of the outer tube taken from the line 22-22 of Figure 21;

Figure 23 is a longitudinal section of the inner tube;

Figure 24 is a bottom view of the inner tube taken on the line 24-24 of Figure 23;

Figure 25 is a front elevation of yet another modification of the invention showing the device in a locked position for attachment to a straight tube having an occluding septum;

Figure 26 is a section taken on the line 26-26 of Figure 25;

Figure 27 is a section taken on the line 27-27 of Figure 26, showing the device in the locked position;

Figure 28 is a section taken on the line 28-28 of Figure 27;

Figure 29 is a section taken on the line 29-29 of Figure 27;

Figure 30 is an exploded view of the unlocked modified coupling device in section showing it released from the septum and tube, with its gripping fingers extended outwardly;

Figure 31 is a front plan view of the inner tube of the modified assembly; and

Figure 32 is a bottom upward-looking view of the inner tube in the position of Figure 31.

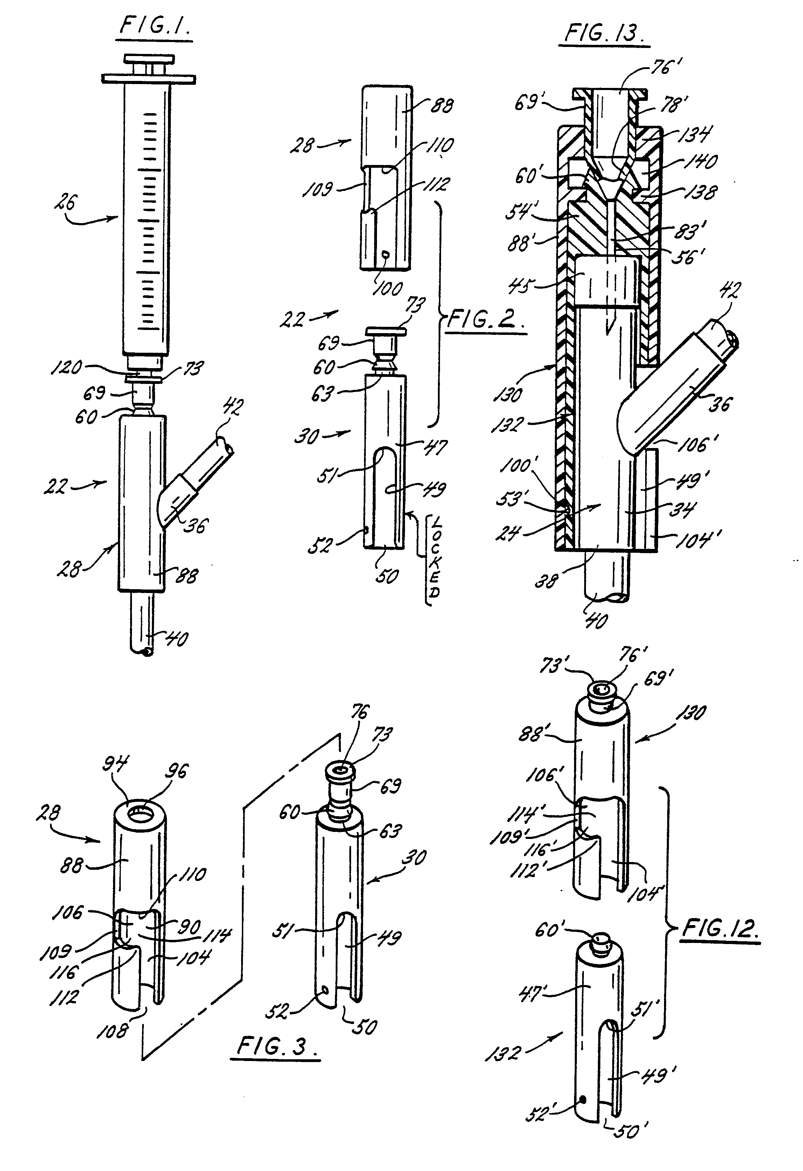

An embodiment of the novel coupling device 22 is shown in Figures 1-11. In Figure 1, the device 22 is shown mounted to a junction tube 24, with a source of fluid for administration to a patient, shown in the form of a syringe 26, being connected to the device 22.

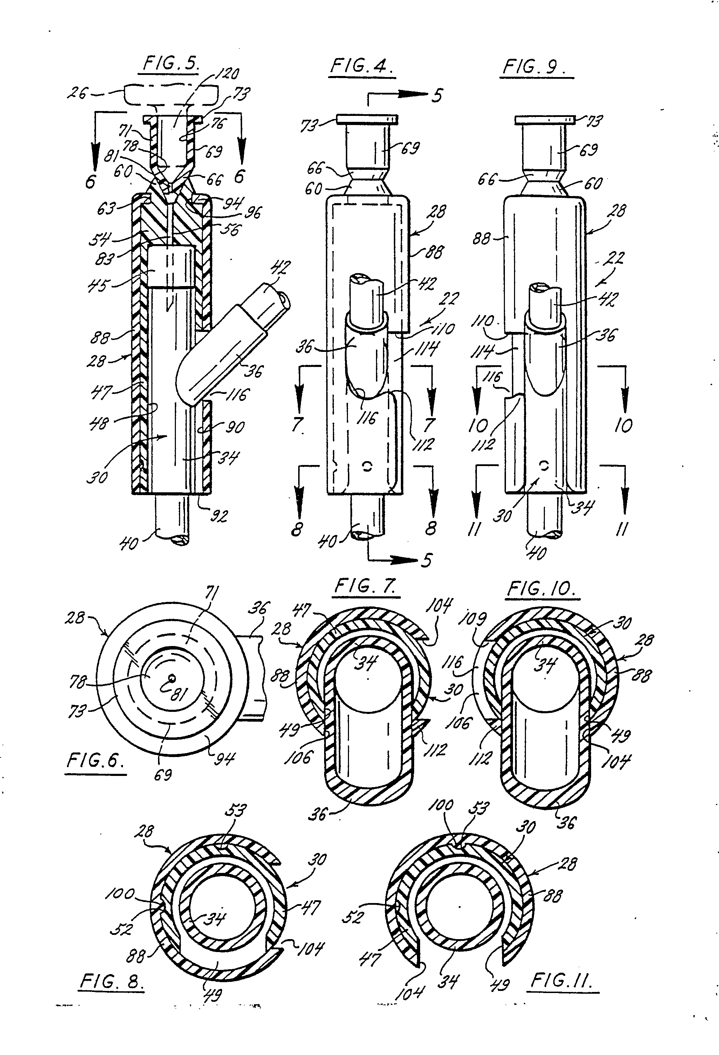

As shown clearly in Figures 2 and 3, coupling device 22 comprises an outer mount tube 28 and an inner mount tube 30. The outer tube 28 can be mounted about the inner tube 30, with both tubes extending about the junction tube 24 in the unlocked position, such as depicted in Figure 9, and in a locked position, such as depicted in Figures 1, 4, and 5.

First, for a better understanding of the operation of device 22, the junction tube 24 is described. Junction tube 24 is of the standard "y" shape, as is known in the art. Junction tube 24 has a main tubular trunk 34 with an angular tubular arm 36 integrally secured thereto. Trunk 34 and arm 36 are both hollow and in fluid flow connection with one another. The distal end 38 of trunk 34 receives a flexible plastic tube 40 which extends to a terminal (not shown) for connection to a catheter (not shown). Such a catheter can extend to fluid flow connection with a patient, as known in the art. The outer end of arm tube 36 has a flexible plastic tube 42 inserted therein. Tube 42 can be connected to a reservoir of fluid (not shown) for intravenous administration, as known in the art. The proximal end of trunk tube 34 is occluded by a penetrable, self-sealing resilient septum 45, as known in the art.

With reference now to inner mount tube 30, there is a main cylindrical section 47 having an inner bore 48. Section 47 has an elongated slot 49 extending from its distal opening 50 proximally toward a closed curved end 51. Section 47 has nears its distal end an exterior pair of recessed dimples 52 and 53. Dimples 52 and 53 receive a nub from outer tube 28 as will be described.

Cylindrical section 47 extends proximally toward a thicker proximal section 54. A smaller cylindrical bore 56 extends through section 54 and opens into bore 48 (as seen in Figure 5). From section 54, tube 30 extends proximally into a frusto-conical lug 60. Lug 60 has an annular groove 63 about its base to receive a locking rim of the outer tube 28, as will be described.

Lug 60 has a proximal funnel-shaped recess shaped to receive and hold as by heat bonding, or by adhesive a frusto-conical portion 66 of a connecting conduit section 69. Conduit section 69 has a main cylindrical portion 71 which extends proximally from portion 66. Portion 71 terminates with an outwardly projecting annular flange 73. Section 69 has a proximal cylindrical bore 76 which extends distally into a conical bore 78. Bore 78 in turn extends distally into a small cylindrical bore 81 that passes through portion 66.

A stainless steel needle 83 has its proximal end fitted securely within a bore at the tip of frusto-conical portion 66, so that the hollow bore of needle 83 is in fluid flow connection with bore 81. Needle 83 telescopically extends through the bore 56 of mount tube section 54. As illustrated in Figure 5, when inner mount tube 30 is mounted to the junction tube 24, needle 83 penetrates septum 45 and extends within the proximal end of junction trunk tube 34.

Hence, it can be seen that a liquid flow conduit is established through the bores 76, 78 and 81 of connecting section 69, and through the bore of needle 83, to permit liquid to flow from the syringe 26 into trunk tube 34.

Now I turn to a description of the outer tube 28. Outer tube 28 comprises a main cylindrical body 88 which has a distal opening 92. At its proximal end, body 88 has an inwardly extending annular rim 94. A cylindrical bore 96 passes through rim 94 to connect with main bore 90. As can be seen in Figure 5, when outer tube 28 is mounted to inner tube 30, the locking rim 94 fits within the annular groove 63 beneath lug 60.

Near the lower end of section 88 is an inwardly projecting nub 100 sized to be lockingly but releasably inserted in either one of the dimples 52 or 53 of the inner tube 30.

Outer tube section 88 has an b-shaped slot comprising a longitudinal slot section 104 and a transverse slot section 106. Slot section 104 has a distal opening 108, while slot section 106 terminates in a longitudinal edge 109. The b-shaped slot having sections 104 and 106 has a transverse proximal edge 110. At the distal juncture of slot sections 109 and 106, body 88 has a proximally projecting nib 112. The distance from the proximal end of nib 112 to the closest point of transverse edge 112 is slightly less than the longitudinal section length of junction tube arm 36 at the point of intersection of those parts when the mount tubes 28 and 30 are connected to junction tube 24 as will be described. Since nib 112 is made of a resilient material, it gives way under arm 36 when the mount tube 28 and junction tube 24 are rotated relative to each other by the hands in either direction. Transverse section 106 is therefore divided into two portions, a narrowed portion 114 and an enlarged portion or hollow 116. The hollow 116 is sized to accommodate the length of the major axis of the elliptical section of the arm 36 at the point wherein the arm intersects the body 88 at hollow 112. The hollow is most preferably further of adequate size to receive the different lengths of the major axes of the elliptical sections produced by the variation in branching angles of junction tubes in conventional use.

Mount tube 28 and mount tube 30, including its connecting section 69, can be made of plastic such as polypropylene, or other selected materials. The outer mount tube 28 in particular should be made of a flexible resilient material, such as plastic, so that the nib 112, nub 100 and the proximal lock rim 94 can compress and flex to operate as desired. Although the connecting section 69 has been shown separate from main body 47, body 47 and connecting section 69 can be integral with one another, and molded of the same unitary piece of plastic. Outer tube 28 can be a unitary piece with all the portions integrally connected with each other, and can be of molded plastic.

In assembling the device 22, as it has been described, the connecting section 69 can first be fitted to body 47 with the needle 83 sliding telescopically through bore 56 of proximal section 54, and with the parts secured as before described. The mount tubes 28 and 30 can then be joined by moving inner tube 30 slidingly within bore 90 of outer tube 28, until the outer surface of lug 60 contacts the distal side of lock rim 94.

At this point, inner mount tube 30 can be shoved as by the hands proximally relative to outer tube 28, so that rim 94 flexes in a proximal direction to thus enlarge the opening of bore 96 to allow passage of lug 60. Lug 60 is moved to the point illustrated in Figure 5, so that the lock rim 94 can flex back to the position shown, to thus lock mount tubes 28 and 30 against longitudinal movement relative to each other. In this Figure 5 position, the lock rim 94 fits within the groove 63 to permit rim 94 to rotate about lug 60. When the tubes 28 and 30 are mounted, the outer surface of inner tube body 47 is telescopically received within outer tube bore 90 for snug fitting, but yet the fit allows rotation of the tubes 28 and 30 relative to each other.

The outer tube 28 can be positioned relative to inner tube 30 so that inner tube slot 49 is aligned with the outer tube slot section 104, such as illustrated in Figure 9. Then junction tube 24 can be moved so that the main trunk 34 is longitudinally aligned with inner tube bore 48, and the junction tube arm 36 is aligned with tube slots 49 and 104. Junction tube trunk 34 is slid within inner tube bore 48 to the position shown in Figure 9, so that the upper surface of junction arm 36 contacts the transverse slot edge 110 of outer tube 28. When this occurs, needle 83 pierces septum 45 and enters within junction tube trunk 34.

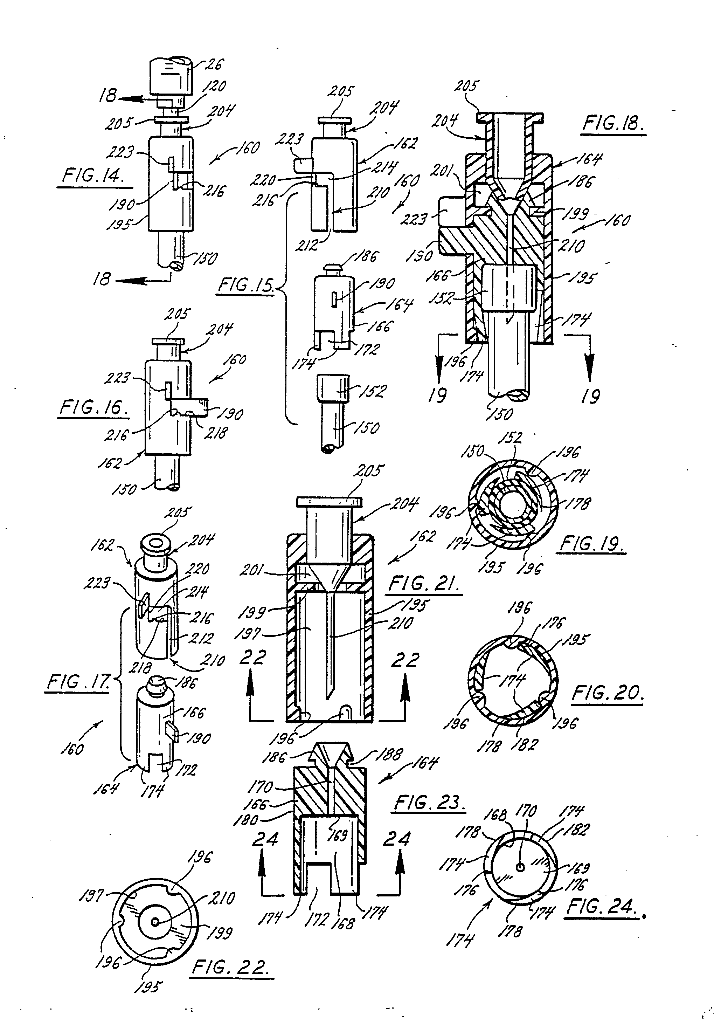

In this Figure 9 position the outer tube nub 100 fits within the dimple 53 (Figure 11). This locks the two tubes 28 and 30 to each other by a force of low resistance so that the tubes are held in fixed position relative to one another. This locking helps to inhibit non-volitional rotation of the two tubes 28 and 30 relative to one another. Yet the lock of nib 100 with dimple 53 is such that tubes 28 and 30 can easily be rotated by the hand relative to each other.

When the two tubes 28 and 30 are mounted to junction tube 24 as shown in Figure 9, device 22 can then be moved to a locked position relative to arm 36. This is done by rotating outer tube 28 counterclockwise relative to junction tube 24 and inner tube 30 (from the view looking at Figure 7) so that junction tube arm 36 slides along b slot edge 110 to compress nib 112 and thence move into the transverse slot 106, such positioning being illustrated clearly in Figures 4 and 5. In this position, inner tube section 47 occludes the outer tube slot section 104. A bright color, such as red, or the word "LOCKED" can be provided on the portion of inner tube section 47 which occludes the longitudinal slot section 104. The bright color or "LOCKED" is visible to an observer through slot section 104 when the locked position is achieved.

In this second locked position, nub 100 is received within the dimple 52 of inner tube 30 (Figure 8). This again helps resist non-volitional rotation of the inner tube 30 relative to the outer tube 28 to help hold the position of Figures 4 and 5. The nub 100 projection which narrows the width of slot section 106 thus provides a gate of resistance for passage of the arm 36 to and from slot sections 106 and 104.

In the locked position of Figures 4 and 5, the arm 36 extends through a window formed by a proximal part of inner tube slot 49, and by the outer tube slot 106. In this position, longitudinal movement of outer tube 28 relative to junction tube 24 is inhibited by the distal and proximal edges of slot section 106. Since inner tube 30 is secured to outer tube 28, inner tube 28 likewise is longitudinally locked.

In the locked position of Figures 4 and 5, transverse movement of arm 36 relative to outer tube 28 is blocked in one direction of rotation by edge 109 of slot section 106. In the other direction of rotation, movement of arm 36 is blocked by the resistance of inner tube section 47 as seen in Figure 7, which in turn is held in position relative to outer tube 28 by the locking of nub 100 and dimple 52, as seen in Figure 8. Rotation is also inhibited by nib 112. Hence there is resistance to movement of arm 36 from slot section hollow 116 in every direction of movement.

With device 22 so mounted to junction tube 24, the needle 83 is securely held within tube 24 in fluid flow connection therewith, so that it cannot be knocked or shaken loose by accident.

In such position, the neck 120 of syringe 26, or a connecting member from another secondary fluid source, can be telescopically inserted within the conforming bore 76 of connector 69. Syringe 26 or other secondary fluid source can then be operated as known in the art to force fluid contained therein through the previously described conduit through needle 83 into junction tube trunk 34, to thus be in fluid flow contact with the liquid in trunk 34 and arm 36. After the liquid has been transferred from syringe 26 into junction tube 24 through needle 83, outer tube 28 can then be hand rotated to align inner tube slot 49 with outer tube slot 104. Such hand rotation releases the lock of nub 100 with dimple 52, and moves nub 100 back to dimple 53 as shown in Figure 11.

Device 22 can then be moved to slide arm 36 through inner tube slot 49 and outer tube slot section 104 to permit complete removal of device 22 from junction tube 24. Device 22 can then be discarded, and junction tube 24 can again be available for receiving a new secondary fluid source.

It can thus be seen that in order to remove arm 36 from slot hollow 116, it is necessary to move outer tube 28 in two different directions relative to arm 36 to move arm 36 from engagement with tube 28. Movement of the tube 28 relative to arm 36 in a single direction, or vice versa, does not disconnect arm 36 from tube 28.

As seen in Figure 5, hollow 116 has a width to receive the arm 36 extending angularly therethrough.

If desired, the slot having two sections 104 and 106 can be provided in the tube 30, and the single slot 49 can be in tube 28.

The connection of tubes 28 and 30 can also be provided by having the recess corresponding to groove 63 located in tube 28, and the locking projection corresponding to rim 94 extending from tube 30.

For a junction tube having an outer diameter of the septum 45 of 7 mm, and an arm 36 extending at an angle of 35°, with the outside diameter through the cylindrical cross section of the arm 36 being 6 mm, the major axis of a longidutinal section ellipse of the arm 36 being 10 mm, the dimensions of the device 22 can be as follows: the inner tube 30 has an internal diameter of about 8 mm, a width for slot 49 of about 6.5 mm. the cylindrical body 47 has a length of about 55 mm, and a thickness of .8 mm. The outer tube 28 has a length of about 58 mm. a thickness of .8 mm., the longitudinal slot section 104 has a width of about 6.5 mm, the narrow passage 114 has a width of about slightly less than the major axis of the elliptical section of the arm 36, such as about 8.5 mm, the hollow 116 has a breadth of slightly greater than the major axis of the longitudinal elliptical cross section such as about 10.5 mm. In order to universally accommodate larger septae, the preferred embodiment of device 22 could be slightly larger.

The size of these dimensions can vary with differences in the angle of extension of the arm 36 relative to trunk 34. The more acute the angle of arm 36 relative to trunk 34. the greater the width of the hollow 116 required to accommodate the portion of the arm 36 extending therethrough, and likewise the wider the passage 114.

Because of the combination of the two tubes 28 and 30 and the tolerances achieved thereby, a single design of tubes 28 and 30 can interact with junction arms having a range of angles of extension for arm 36.

For example, the hollow 116 may have a breadth of 11.5 mm and because of the presence of inner tube 30, still securely hold an arm 36 having an angle of extension of 44° and a major axis length of the arm 36 longitudinal section of only 7.5 mm. The ability to securely hold the arm 36 despite considerable differences between such a major axis length and hollow 116 breadth is due to the tightness of fit provided by the inner tube elongated slot 49. This ability provides for the construction of a device 22 which can tightly hold virtually all existing commonly used junctions of the type similar to junction tube 24.

Now I turn to the modification shown in Figures 12 and 13. With this design, the outer tube is shown as 130 and the inner tube is shown as 132. In general, with this modification. the means for receiving the liquid from an outside source, such as a receptacle. is mounted with tube 130, and the conduit flow from the receiving means to within the junction tube 24 also extends with the tube 130. The b-shaped slot in tube 130 can be the same as that described for outer tube 28.

More specifically, outer tube 130 has a main cylindrical body 88'. At the proximal end of body 88' is an inwardly extending annular collar 134. Collar 134 has a cylindrical bore extending therethrough that telescopically and snugly receives a receptacle 69'. Heat bonding or adhesive can be used to hold receptacle 69' within the said bore. Receptacle 69' can be the same as that shown as 69. A needle 83' is mounted with receptacle 69' so that the flow channel of needle 83' is in liquid flow connection with the bores 76' and 78' of receptacle 69'. Distal to the collar 134, a resilient angular ring-shaped rib 138 projects inwardly from tube wall 88'. A cavity 140 is formed between rib 138 and flange 134.

The remaining portion of body 88' is similar to that of body 88. with a b-shaped slot having sections 104' and 106' with a nib 112'. a hollow 116' and a narrowed slot passageway 114', as well as a slot edge 109'.

The inner tube 132 has a body 47' with a longitudinal slot 49', like that of slot 49. having an opening 50' and an end 51'. Body 47' likewise has dimples 52' and 53' corresponding to the dimples 52 and 53 to receive a locking nub 100' (Figure 13).

Tube 132 has an interengaging lug 60' having the same shape as lug 60. with an annular groove beneath it for receiving the annular rib 138, as seen in Figure 13. Tube 132 likewise has a thicker proximal section 54' corresponding to section 54. The needle 83' can pass through a bore 56' during assembly to be described.

In operation, the device 22' can be assembled by aligning the tubes 130 and 132, such as depicted in Figure 12, and moving tube 132 to fit within the bore 90' of tube 130. Tube 132 is moved so that the exterior surface of frusto-conical lug 60' presses against the resilient annular rib 138 to flex it and enlarge the opening within rib 138 to permit passage of lug 60' therethrough. Lug 60' moves within cavity 140 as shown in Figure 13. This allows rotation of tube 132 relative to tube 130, but yet longitudinally locks tube 132 to tube 130.

To mount the device 22' to junction tube 24, slot 49' and slot section 104' are aligned with each other as before described, and the junction arm 36 moved to the point near the proximal edge 51' of slot 49' to the Figure 13 position.

The modification 22' can function similarly to that of the device 22. The outer tube 130 can be rotated relative to the inner tube 132 so that the junction arm 136 moves through the narrow slot passage 114' beyond nib 112' into hollow 116'. In this position, the arm 36 is then bound by the proximal and distal edges of slot section 106' as well as by slot edge 109', and further is confronted by the resistance of nib 112', and the abutment of inner tube 47' against the arm 36 near nib 112' to provide locking engagement. With this position, the arm 36 cannot be disengaged from the device 22' through movement of the arm 36 in a single direction of motion relative to the tube 130.

Rotation of the inner tube 132 can be achieved without rotation of the receptacle 69', in contrast to the design of device 22. This has an advantage in that if the thumb is applied to the arm 36 to force it from slot 104' into the hollow 116', the inner tube 132 moves with the junction tube 24 while the outer tube 130 remains stationary relative to the hand and the junction tube 24. This means that receptacle 69 does not rotate relative to hand movement, and this helps avoid the potential for disengagement of the syringe or other source of fluid, such as a flexible tube, from the receptacle 69′ by the force of the hand. This method of moving the arm into the locking engagement with the thumb is very convenient and therefore appears to be a preferable mode of operation.

To remove the arm 36′ from the locking engagement within hollow 116′, one hand can hold outer tube 130 while the forefinger of the other hand is placed on arm 36 to force it out of hollow 116′ into slot section 104′ to the Figure 13 position. Device 22′ can be completely removed from junction tube 24 by sliding trunk 24 away from the tubes 130 and 132.

The embodiment in Figures 12 and 13 also illustrates how the single member device can operate. In this instance, the tube 130 can be singularly used without the tube 132.

The tube 130, when so used, can be reduced to have an inside diameter such as that shown for the tube 132. The tube 130, with the reduced inside diameter, can then be aligned with junction tube 24 so that the arm 36 can be moved through slot section 104′ to abut slot edge 110′. Arm 36 and tube 130 can then be rotated relative to each other to move arm 36 past nib 112′ into hollow 116′. In this position, movement of arm 36 is again blocked by the proximal and distal edges of slot section 116′ as well as by slot edge 109′. Further, resistance to arm 36 movement is provided by nib 112′. Thus movement in any one direction of motion will not disengage arm 36 from tube 130.

Tube 130 can be disengaged from arm 36 by rotating the two relative to each other to move arm 36 toward the proximal end of slot 104′. Tube 130 can then be slid away from arm 36 to disengage tube 130 from junction tube 24.

The embodiment described using tube 130 as a single member may not fit snugly upon arms which extend at substantially different angles than shown for arm 36 and therefore such a design is less universally applicable and is less preferred than the arrangement 22′ which uses both tubes 130 and 132, and the arrangement 22 which uses tubes 28 and 30, which embodiments can fit snugly upon junction arms which extend at varying angles.

An embodiment comprising only tube 30 is additionally useful for engaging junction tube trunk 34 and arm 36 for administration of fluid when a secure locking means is not desired.

Figures 14-24 show a modification of a coupling device for use with intravenous tubing systems which do not have a "Y" shaped junction section. A straight tube 150 has at its proximal end a connection terminal 151 comprising a penetrable self-sealing septum 152. A thin wrap (not shown) can extend about tube 150 and septum 152 to secure them together.

The modified coupling device 160 comprises an outer tube 162 and an inner tube 164. Generally, the outer tube 162 is mounted about inner tube 164. The two tubes rotate relative to each another as they fit about septum 152 and part of tube 150, to be in locked and unlocked positions.

The inner tube 164 comprises a main cylindrical section 166 having at its lower end a larger bore 168 having a proximal wall 169. Bore 168 extends proximally into a smaller bore 170. The distal end of cylindrical section 166 has three slots 172 formed therein to form three gripping fingers 174 therebetween. As seen in Figures 19, 20 and 24, the three fingers 174 are tapered circumferentially so that they each have a thicker wall section 176 which tapers into a thinner wall section 178. The cylindrical section 166 has an exterior surface 180, with the portions 182 of that surface forming the exterior surface of the fingers 174.

At the proximal end of cylindrical section 166 extends an integral frusto-conical lug 186, which is shaped like and functions like lug 60 described earlier. Lug 186 likewise has an annular groove 188 extending about its base, which corresponds to groove 63. Extending radially and integrally from section 166 is a rotation arm 190.

I refer now to outer tube 162. Tube 162 comprises a principal cylindrical section 195 having a distal bore 197 which opens at the distal end of section 195. At the distal end of section 195, three knobs 196 project integrally into bore 197. Knobs 196 interact with the fingers 174 as will be described. An annular rib 199 extends integrally from cylindrical section wall 195 so that a cavity 201 is formed proximally thereto. Rib 199 and cavity 201 correspond to rib 138 and cavity 140 illustrated in Figure 13.

At its proximal end, cylindrical section 195 has a longitudinal bore which telescopically receives a connecting conduit section 204 with connector flange 205, section 204 being of the same shape as the conduit section 69 illustrated in Figures 1-13. Section 195 can be held thereto as by an adhesive. Alternatively, section 204 can be integral with cylindrical section 195.

A longitudinally extending hollow needle 210 is connected to section 204 in the manner heretofore described in Figures 1-13, so that liquid flow is established through needle 210 into the bores of connector 204.

Outer tube cylindrical section 195 has a generally L-shaped slot 210 formed therein. Slot 210 has a longitudinal section 212 which connects with a horizontal slot section 214 of greater width. A locking nib 216 extends proximally from the distal edge 218 of slot 214. Slot 214 has a longitudinal closed end 220. A longitudinal space is formed between nib 216 and slot end 220 sufficient to receive the width of the inner tube lock arm 190. Extending integrally and radially from cylindrical section 195 is a gripping arm 223.

Inner tube 164 can be rotatably mounted to outer tube 162 by moving it upwardly from the position of Figure 23 toward the outer tube as it is shown in Figure 21. The frusto-conical section 186 extends through the bore of annular rim 199 which can flex so that section 186 passes proximally beyond rim 199. Rim 199 then extends into the annular groove 188 to lock against longitudinal movement but to permit rotation. The funnel-shaped portion of section 186 receives the conical distal section of connecting section 204 as heretofore described. In such assembling of the device, the inner tube arm 190 slides through outer tube slot section 212.

After frusto-conical section 186 is engaged, the device 160 can be mounted about septum 152 and tube 150. This is done by longitudinally aligning device 160 with tube 150. Inner tube arm 190 is positioned toward the side of longitudinal slot 212, to be in the unlocked position. In this unlocked or open position, the fingers 174 are positioned relative to knobs 196 as shown in Figure 16. In such position, the fingers 174 are spaced outwardly so that their outer surfaces 182 abut the inner surface of tube bore 197. Septum 152 can pass through the opening between fingers 174, so that the proximal surface of septum 152 abuts the end 169 of bore 168, with needle 210 piercing septum 152.

Then the human hand can press the outer tube arm 223 and the inner tube arm 190 together to rotate tubes 162 and 164 relative to each other. As this occurs, arm 190 moves through slot 214 until it reaches nib 216, then compresses nib 216 to pass beyond it to rest against slot edge 220, as illustrated in Figure 14. In this position, the two arms 190 and 223 are closely positioned relative to one another.

As the rotation from Figure 16 to Figure 14 occurs, the inner tube fingers 174 are rotated clockwise (as viewed looking at Figure 20) so that the smaller tapered ends 178 of the fingers first ride upon the knobs 196. Such movement occurs until the fingers 174 reach the position of Figures 18 and 19, in which the knobs 196 press against the thicker finger portions 176. This in turn has caused the fingers 174 to move inwardly from their Figure 20 position so that they now grip about tube 150 at a position distal to the septum 152. As a result, any distal movement of the septum 152 and tube 150 relative to the device 162 is met by the gripping resistance of the fingers 174 about septum 152.

In the locked position, the inner tube lock arm 190, and hence inner tube 160, is held against movement relative to outer tube 162 by virtue of the contact of the proximal and distal sides of slot 214 against the corresponding proximal and distal edges of arm 190, as well as from the resistance of slot edge 220 and nib 216. This helps to prevent non-volitional disengagement from the locked position. Movement in three directions is totally blocked by the edges of slot 214, and movement in the fourth direction is blocked by the lower resistance of nib 216. Hence, there is resistance to movement of the arm 119 from its locked position of Figures 14 and 18, in every direction.

To release the lock of tubes 162 and 164, the human fingers press lock arms 190 and 223 away from one another, so that arm 190 compresses nib 216 to ride over it, to return to the position of Figures 16 and 20. The lock fingers 174 have then moved outwardly away from septum 152 to no longer grip tube 150 beneath septum 152. Hence, in the Figures 16 and 20 positions, the device 160 can be moved longitudinally to disengage from septum 152 and tube 150.

Outer tube cylinder 195 can be of a flexible plastic such as polypropylene so that the nib 216 can flex just as nib 112 and 112' flex in the operation of the Figures 1-13 embodiments. Inner cylinder 166 preferably is of flexible and compressible plastic such as polyvinyl chloride so that the gripping fingers 174 can flex inwardly to grip about the septum 152 and so that the fingers 174 can be compressed against tube 150 by knobs 196.

In the locked position, the neck 120 of syringe 26, or a connecting member from another secondary fluid source, can be telescopically inserted within the bore of the connector 204 in the same fashion heretofore described for connector 69. The gripping action of the locked device 160 resists disengagement caused by pulling connector 204.

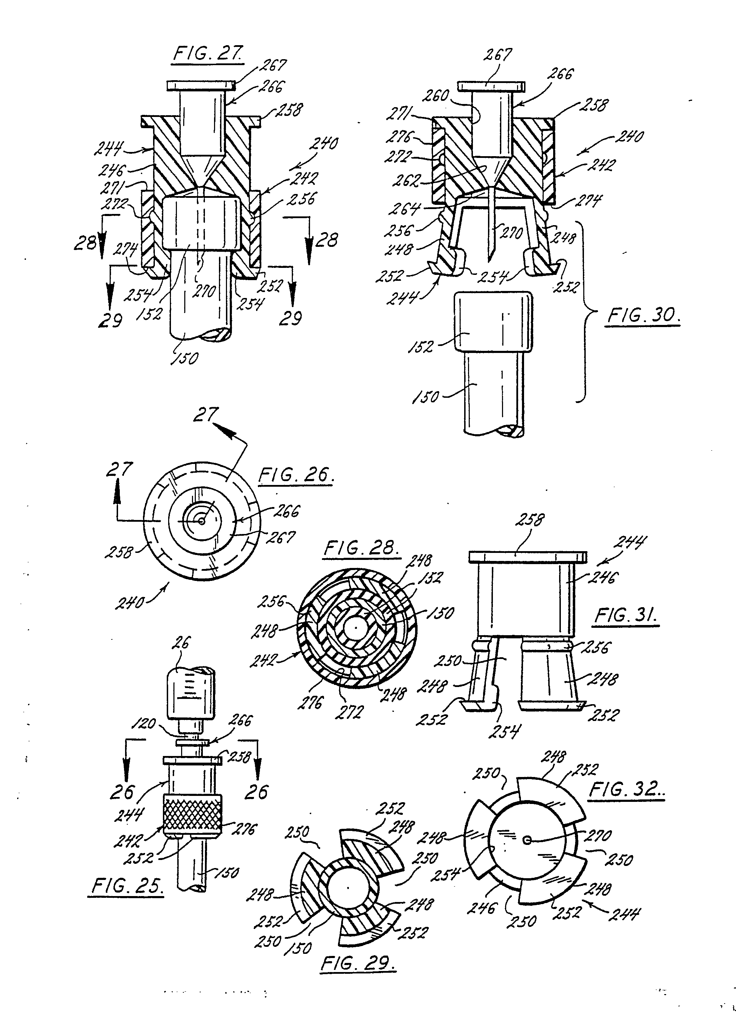

Now reference is made to the modification of Figures 27-32. The modified coupling device 240 is shown gripping about a septum 152 attached to a tube 150 by tape 154. Device 240 comprises an outer tube 242 and an inner tube 244. Inner tube 244 comprises a cylindrical section 246 which has three depending integral gripping fingers 248 with slots 250 formed therebetween. As seen in Figures 31 and 32, fingers 248, when not compressed by tube 242, flare or taper outwardly. At the proximal end of each finger 248 is an outwardly projecting stop ledge 252, and an inwardly projecting lock lug 254. Toward the proximal end of each finger 248 is an outwardly projecting ridge 256.

At the proximal end of cylindrical section 246 is an integral annular stop flange 258. As shown in Figure 27, section 246 has at its proximal end a cylindrical bore 260 which extends into a funnel-shaped bore 262. Bore 262 opens into a tapered bore 264 at the distal end of section 246. Bores 260 and 262 receive, as by a telescopic fit and hold as by adhesive, a connecting section 266 with connector flange 267 which corresponds to the connecting sections heretofore described, such as 204. A hollow longitudinally extending needle 270 passes through bore 264 into a receiving bore of connector tube 266, as heretofore described, so that liquid flow is established through the needle 270 and the bores in connector 266.

The outer tube or sleeve 242 can be mounted to tube 244 by pressing the fingers 248 inward and sliding tube 242 distally past the ledges 252 so that tube 242 passes to the position of Figure 30. In such position, the proximal edge 271 of tube 242 abuts the distal side of stop flange 258. Tube 242 has an inner annular recess 272, as well as a lower cylindrical end 274. Tube 242 has an outer gripping surface 276 which can have cross-hatched gripping grooves such as seen in Figure 25.

In operation, the device 240 can be moved from the position relative to septum 152 shown in Figure 30, so that the septum 152 is received within the three gripping fingers 248. As this occurs, needle 270 punctures septum 152 and enters tube 150. Then outer tube 242 can be slid distally to the position of Figure 27. As this occurs, the rigid tube 242 presses the three fingers 248 inwardly, and the ridge 256 is compressed. In the fully locked position of Figure 27, outer tube 242 holds the fingers 248 so that their lock lugs 254 grip beneath septum 152. In this position, inner tube ridge 256 extends into outer tube recess 272 to hold outer tube 242 in the locked position. In such position, the outer tube end 274 abuts the stop ledge 252 to resist any farther distal movement of tube 242.

The neck 120 of syringe 26, or another member from some other secondary fluid source, can be telescopically inserted within the bore of connector 266 (or for more secure connection can be threadingly received onto flange 267), and liquid discharged therethrough through needle 270 into tube 150. Should any pulling force through the secondary fluid source be exerted on the connector 266 or inner tube 244, the gripping action of the finger lugs 254 will resist disengagement from tube 150 and septum 152.

To release the grip of fingers 248, outer tube 242 is slid proximally back to the position of Figure 30. Such hand movement overcomes the lock of ridge 256 in recess 272, so that the ridge 256 is compressed to allow such movement.

Inner tube 244 is of resilient material, such as molded plastic, such as polyvinyl chloride. The resiliency allows the ridge 256 to give way during the sliding movement of outer tube 242 and for lugs 254 to compress against tube 150 when outer tube 242 is slid to the distal position of Figure 25. Outer tube 242 is preferably of more rigid material such as polycarbonate to facilitate the flexing action of fingers 248.

An important advantage of the modification is its inordinate ease of operation. Often a secondary different fluid source (not shown) other than a syringe may be connected to a long flexible conduit (not shown) which can be securely attached to connector 266 (as by threading onto connector flange 267). The long flexible conduit can be easily and securely locked and unlocked about the septum 152 by simply grasping the outer gripping surface 276 with the fingers and thumb and advancing the inner tube 244 over the septum 152. When the septum 152 engages the wall of bore 264 of the inner tube 244, additional advancing force will cause the outer tube 242 to slide distally over the inner tube 244 until it abuts the ledges 252. The gripping fingers 248 in this position are compressed against the tube 150. In this position, the secondary fluid source cannot be disengaged from the septum 152.

Disengagement cannot be achieved even by significant force pulling upon this secondary fluid source or its associated conduit, since such a pull would extend through connector 266 to inner tube 244, which is locked upon tube 150 by compressed fingers 248. Therefore, inadvertent disconnection due to accidental and even strong pulling forces upon the secondary fluid source or its associated conduit is prevented. However, a much less forceful pull by the thumb and fingers against outer tube gripping surface 276 will cause the outer tube to slide proximally along the inner tube 244 until it strikes the stop flange 258.

This allows the inner tube 244 to disengage from about the septum 152. Therefore, simple pushing and pulling forces against the gripping surface 276 of the outer tube 242 allows easy engagement and disengagement, but even strong inadvertent forces against the attached secondary fluid source or its associated conduit (not shown) will not cause disengagement.

Another important advantage is that locking can be achieved by a single hand. Therefore, the nurse can stabilize the tube 150 with one hand. With the fingers and thumb on gripping surface 276, the nurse can advance the device 240 over the septum 152 and lock about the septum 152 with one advancing maneuver.

The outer tube 242 can have a length of 15 mm. with an internal bore diameter of 11 mm. The inner tube can have a length of 34 mm. The distance between opposing projecting lugs 254 in the flared and open position of Figure 30 can be 11 mm., while the distance between lugs 254 in the locked position of Figure 27 can be 7.5 mm. The lugs 254 can have a thickness of 2.5 mm. The lugs 254 are preferably of compressible material so that they can be compressed between the rigid tube 150 and the rigid outer tube 242 within the locked position of Figure 27.

Bore 197 can be 12.5 mm. in diameter. Knobs 196 can have a projecting width of 1 mm. into bore 197. The thicker wall section 176 of fingers 174 can be 1.8 mm. and the thinner wall section 178 can be 1 mm. in width.

There are various changes and modifications which may be made to the invention as would be apparent to those skilled in the art. However, these changes or modifications are included in the teaching of the disclosure, and it is intended that the invention be limited only by the scope of the claims appended hereto.

## Claims

1. A medical connector for connecting two fluid conveying conduits, comprising a first connecting portion (244) adapted to be connected to a first fluid conveying conduit (150) having a septum (152) at an end thereof, and a second connecting portion (266) adapted to be connected to a second fluid conveying conduit (120),
characterised in that the second connecting portion (266) has one end adapted to be connected to the second fluid conveying conduit (120) and has at its other end a fluid conveying needle (270), and in that the first connecting portion (244) defines a space into which the needle (270) extends towards an open end of the space and which is adapted to receive the septum (152) of the first fluid conveying conduit (150) so that the needle penetrates the septum, the first connecting portion (244) including flexible gripping means (248) extending towards the said open end of the space and bounding the space, and there being providing locking means (242) manually moveable from a first position in which the septum (152) may be inserted into the said space through the said open end to be penetrated by the needle (270), to a second position where the locking means (242) flexes the gripping means (248) inwardly to trap and lock the septum (152) in the space.

2. A connector according to claim 1 in which the flexible gripping means comprise flexible fingers (248) extending towards the open end of the said space.

3. A connector according to claim 1 or 2 in which the locking means (242) extends around the first connecting portion (244) and is slidable on the first connecting portion (244).

4. A connector according to claim 2 or 3 in which the needle (270) is positioned in the said space at a location such as to reduce accessibility to manual contact by an operator.

5. A universal medical connector for manually connecting a first fluid conveying conduit (150) in fluid connection with a patient's vasculature, and having a septum (152) at an end thereof, to a second fluid conveying conduit (120) in fluid connection with a source (26) of fluid for administration to a patient, and having an open end thereof, comprising:
means defining a needle hub having one end adapted to couple to said open end of said second fluid conveying conduit (120), the other end adapted for connection to a fluid conveying needle (270), and a bore extending therebetween for conveying fluid from an open ended conduit (120) coupled to said one end to a needle (270) connected to said other end;
a single needle (270) mounted to said other end of said needle hub;
means (244) defining a space through which said needle (270) extends toward an open end thereof and adapted for receiving said septum (152) of said first fluid conveying conduit (150) so that said needle penetrates said septum and fluid flows through said needle (270) into said first conduit (150), said needle being positioned in said space so as to be inaccessible to the fingers of a person connecting said conduits (120 and 150), said defining means (244) further including flexible fingers (248) extending toward said open end of said space and bounding said space; and
locking means (242) mounted about said space defining means (244) and manually moveable from a first position in which said septum (152) may be inserted into said space through said open end to be penetrated by said needle (270) to a second position flexing said fingers (248) inwardly to trap and lock said septum (152) in said space.

6. A connector according to claim 5 wherein said one end of said needle hub is provided with a flange (267) surrounding the opening of said bore so that an open end of a second conduit (120) can be releasably received on said flange (267) or releasably received into said opening of said bore.

7. A connector according to claim 5 or 6 wherein said space defining means (244) is a inner tube of molded plastic and said locking means (242) is an outer tube of plastic and is more rigid than said inner tube (244).

8. A connector according to claim 7 wherein said outer tube (242) is slidable along said inner tube (244) in the direction said needle (270) extends.

9. A connector according to claim 7 or 8 wherein said inner tube (244) is provided with separated stop surfaces (252) limiting the sliding movement of said outer tube (242).

10. A connector according to any of claims 5 to 9 wherein said finger (248) has a thickened portion (254) adjacent the end thereof which flexes inwardly to lock said septum (152) in said space.

FIG.1.

FIG.2.

FIG.13.

FIG.3.

FIG.12.

FIG.5.

FIG.4.

FIG.9.

FIG.6.

FIG.7.

FIG.10.

FIG.8.

FIG.11.

FIG.14.

FIG.15.

FIG.16.

FIG.17.

FIG.18.

FIG.19.

FIG.20.

FIG.21.

FIG.22.

FIG.23.

FIG.24.

FIG. 27.

FIG. 30.

FIG. 26.

FIG. 28.

FIG. 31.

FIG. 25.

FIG. 32.

FIG. 29.